# EUROPÄISCHE PATENTSCHRIFT

(11) **EP 4 474 000 B1**
(45) Veröffentlichungstag und Bekanntmachung des Hinweises auf die Patenterteilung: **06.08.2025**
(21) Anmeldenummer: 24179561.6
(22) Anmeldetag: 03.06.2024
(51) Int. Cl.: A61M 60/109, A61M 60/279, A61M 60/38, A61M 60/441

(54) **AUFSTECK- UND SICHERUNGSVORRICHTUNG DES ROTORS EINER PERISTALTISCHEN BLUTPUMPE**
ANTI-SAGGING AND SECURING DEVICE FOR THE ROTOR OF A PERISTALTIC BLOOD PUMP
DISPOSITIF DE FIXATION ET DE FIXATION DU ROTOR D'UNE POMPE À SANG PÉRISTALTIQUE

(30) Priorität: 07.06.2023 DE 102023115059
(43) Veröffentlichungstag der Anmeldung: 11.12.2024
(73) Patentinhaber: B. Braun Avitum AG, 34212 Melsungen (DE)
(72) Erfinder: HORSCHKE, Sebastian, 37235 Hessisch Lichtenau (DE)
(74) Vertreter: Winter, Brandl - Partnerschaft mbB

(56) Entgegenhaltungen:
- WO-A1-2007/080499
- US-A- 4 861 242
- US-A- 5 062 775
- US-B2- 7 547 200

## Beschreibung

### Technisches Gebiet

Die Offenbarung betrifft eine peristaltische Blutpumpe für ein medizinisches Gerät zur extrakorporalen Blutbehandlung. Derartige Blutpumpen haben ein Pumpengehäuse mit einer gebogenen bahnartigen Lauffläche und einen innerhalb der Lauffläche drehbaren Rotor, wobei ein Schlauchsegment zwischen die Lauffläche und dem Rotor eingelegt ist. Der Rotor ist mit einer Wellenaufnahme auf einer Antriebswelle der Blutpumpe anbringbar, und es ist ein Verriegelungselement zur axialen Sicherung vorgesehen. Derartige peristaltische Pumpen werden auch als Schlauchrollenpumpen bezeichnet.

Die vorliegende Offenbarung betrifft konkret die Antriebswelle und die Aufsteck- und Sicherungsvorrichtung des Rotors einer derartigen peristaltischen Blutpumpe.

### Hintergrund der Erfindung

In medizinischen Geräten zur extrakorporalen Blutbehandlung (Dialyse) werden oftmals peristaltische Schlauchrollenpumpen (siehe z.B. US7547200B2) eingesetzt, welche das entnommene Blut des Patienten zu einem Dialysator und zum Patienten zurückfördern. Dabei liegt ein kreisförmig gekrümmtes Schlauchsegment mit seiner Außenseite an einer entsprechend gekrümmten inneren Lauffläche des Pumpengehäuses an. Ein innerhalb des Schlauchsegments liegender Rotor der Blutpumpe bewegt sich dann mit daran angebrachten Rollen entlang dem Schlauchsegment, wobei die Rollen den Schlauch lokal radial nach außen eindrücken und so mit den elastischen Materialeigenschaften des Schlauchs eine Blutförderung durch den Schlauch erzeugen. Dafür wird das Blut dem Schlauch über einen ersten Anschluss zugeführt und über einen weiteren Anschluss am anderen Ende des Schlauchs wieder abgeführt. Der Schlauch bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird.

### Stand der Technik

Aus der EP 2 682 604 A1 ist eine peristaltische Blutpumpe bekannt. Die Übertragung des Drehmoments von der Antriebswelle zum Rotor erfolgt durch einen Formschluss. Weiterhin ist der Rotor von der Antriebswelle abziehbar, damit der Schlauch gewechselt werden kann. Dazu ist der Rotor auf der Antriebswelle mit einem ebenfalls formschlüssigen Schnappmechanismus bzw. Rastmechanismus axial gesichert. Ein axial wirkendes Verriegelungselement ist mittels eines manuellen und gefederten Druckschalters bewegbar, wodurch der Rotor freigegeben wird und axial abgezogen werden kann. Der Schalter ist so positioniert, dass er beim Ergreifen des Rotors durch das Bedienpersonal der Blutpumpe bzw. des Dialysegerätes gleichzeitig niedergedrückt werden kann.

Das Aufsetzen des Rotors auf die nicht rotationssymmetrische Antriebswelle ist für das Bedienpersonal in den weitaus meisten Fällen mit einem gefühlvollen Folgen einer Drehbewegung des Rotors verbunden, die von der Antriebswelle erzwungen wird. Diese erzwungene Drehbewegung des Rotors stellt für das oftmals unter Zeitdruck stehende Klinikpersonal jedes Mal eine kleine Herausforderung an Geschicklichkeit und/oder Geduld dar.

Dieser Nachteil ist behoben bei einer unter dem Namen Dialog+ der Anmelderin bekannten peristaltische Blutpumpe, die eine rotationssymmetrische Antriebswelle hat, wodurch die erzwungene Drehbewegung beim Aufstecken des Rotors entfällt. Die Übertagung des Drehmoments von der Antriebswelle auf den Rotor erfolgt in Betrieb der Blutpumpe über einen umfänglichen Reibschluss am kreiszylindrischen Anlagebereich des Außenmantels der Antriebswelle, womit auch ein Freilauf gebildet ist. Um den Rotor von der Antriebswelle zu lösen, muss ein Schalter gedrückt werden, der an der Oberfläche des Rotors angeordnet ist, und dessen Betätigungsfläche bündig mit der dortigen Gehäusewand des Rotors abschließt. Die Betätigungsfläche des Schalters und die dortige Gehäusewand sind senkrecht zur Antriebswelle angeordnet. Daher kann beim Greifen des Rotors durch das Bedienpersonal nicht gleichzeitig mit demselben Finger der Schalter betätigt werden, mit dem auch der Rotor gegriffen und abgezogen wird.

Bei der unter dem Namen Dialog+ der Anmelderin bekannten peristaltische Blutpumpe sind Rotor und das Schlauchsegment in einem schüsselartig ausgeformten Bereich der Blutpumpe angeordnet, in dessen Mitte zapfenartig die rotationsymmetrische Antriebswelle aus dem Boden herausragt. Die Antriebswelle hat einen Kopf und eine umlaufende Nut, die von einer bodenseitigen geneigten Flanke und von einer kopfseitigen Flanke mit maximaler Steilheit begrenzt ist. In anderen Worten hat die kopfseitige Flanke der Nut die Form eines Kreisrings, der senkrecht zur Mittelachse der Antriebswelle ausgerichtet ist. An dieser kopfseitigen Flanke stützt sich ein Verriegelungselement ab, wenn der Rotor mit einer Kraft weg vom Boden in Abzugsrichtung des Rotors beaufschlagt wird, und wenn nicht gleichzeitig auf die Betätigungsfläche des Schalters gedrückt wird.

Am Kopf im Übergang zwischen einem kreiszylindrischen Mantel und einer kreisscheibenförmigen Stirnfläche ist eine Verrundung vorgesehen, die zum leichteren Aufstecken des Rotors dient.

### Kurzbeschreibung der Erfindung

Aufgabe der vorliegenden Offenbarung ist es, eine Antriebswelle für eine peristaltische Blutpumpe und eine Rotationseinheit bestehend aus einer Antriebswelle und einem Rotor für eine peristaltische Blutpumpe zu schaffen, bei denen ein Aufstecken des Rotors weiter vereinfacht ist.

Diese Aufgabe wird in Hinblick auf die Antriebswelle mit der Merkmalskombination des Anspruchs 1 und im Hinblick auf die Rotationseinheit mit der Merkmalskombination des Anspruchs 10 gelöst.

Die Antriebswelle gemäß der Offenbarung ist zur Aufnahme durch Aufstecken eines Rotors einer peristaltischen Blutpumpe eingerichtet und ausgelegt. Die Antriebswelle erstreckt sich entlang ihrer Mittelachse und weist an einem freien Endabschnitt einen rotationssymmetrischen Kopf auf, der von einer - entlang der Mittelachse betrachtet benachbarten - Nut begrenzt ist. An dem Kopf ist ein durch eine Fase gebildeter konischer oder kegelstumpfförmiger Gleitabschnitt angeordnet. Damit ist die Montagekraft des Rotors gering. Damit ist auch eine Einführhilfe für den Rotor geschaffen, die einen axialen Versatz und eine nicht senkrechte Aufsteckbewegung toleriert und ein Verhaken des Rotors vermeidet.

Vorzugsweise erstreckt sich der Gleitabschnitt entlang der Mittelachse der Antriebswelle betrachtet über 30 bis 40%, vorzugsweise 35% der Axialerstreckung (der Länge) des Kopfes.

Vorzugsweise hat der Gleitabschnitt einen Winkel in einem Bereich von 5° bis 20°, bei einem konkreten Ausführungsbeispiel 15° zur Mittelachse der Antriebswelle.

An der von der von der Nut abgewandten Seite der Fase bzw. des Gleitabschnitts kann die komplette Stirnseite der Antriebswelle ballig gestaltet sein. Alternativ kann der Kopf der Antriebswelle eine Stirnfläche aufweisen, wobei ein Übergang von der Fase bzw. dem Gleitabschnitt zu der Stirnfläche verrundet ist mit einem Radius vorzugsweise von größer als 1 mm, bei einem konkreten Ausführungsbeispiel 1,5 mm. In beiden Fällen ist das Aufstecken des Rotors weiter vereinfacht und ein Verhaken vermieden.

Zwischen der Fase bzw. dem Gleitabschnitt und der Nut ist vorzugsweise ein (per se aus dem Stand der Technik bekannter) kreiszylindrischer Kopfabschnitt vorgesehen, der sich - entlang der Mittelachse der Antriebswelle betrachtet - vorzugsweise über 15 bis 25%, bei einem konkreten Ausführungsbeispiel 20% der Axialerstreckung (der Länge) des Kopfes erstreckt.

Ein Übergang von dem Gleitabschnitt zu dem kreiszylindrischen Kopfabschnitt ist vorzugsweise verrundet mit einem Radius vorzugsweise zwischen 2 bis 6 mm, bei einem konkreten Ausführungsbeispiel 4 mm. Damit ist das Aufstecken des Rotors weiter vereinfacht und ein Verhaken vermieden.

Mit den vorbeschriebenen Ausgestaltungen der Antriebswelle kann ein tastenloses Aufstecken des Rotors auf relativ kleinem Bauraum realisiert werden.

Vorzugsweise ist ein Übergang von dem kreiszylindrischen Kopfabschnitt zu der Nut verrundet mit einem Radius vorzugsweise zwischen 0,1 bis 0,3 mm, bei einem konkreten Ausführungsbeispiel 0,25 mm. Diese Verrundung dient hauptsächlich einem einfacheren Entnehmen des Rotors, damit dieser während der Entnahme nicht so leicht verhakt. Die Verrundung ist vergleichsweise klein, damit kein großer Einfluss auf den Hinterschnitt des Verriegelungselements entsteht.

Bei einer bevorzugten Weiterbildung der Antriebswelle ist an einer von dem Kopf abgewandten Seite der Nut ein kreiszylindrischer Anlagebereich einer oder für eine reibschlüssige Kupplung gebildet. Eine zwischen einem Nutgrund der Nut und dem kreiszylindrischen Anlagebereich angeordnete antriebsseitige Nutflanke ist konisch oder kegelstumpfförmig zur Mittelachse schräg gestellt. Ein Übergang von der Nutflanke zu dem kreiszylindrischen Anlagebereich ist verrundet mit einem Radius vorzugsweise zwischen 1,5 bis 4 mm, bei einem konkreten Ausführungsbeispiel 2 mm. Damit wird erreicht, dass die Passungen im Rotor, die als Lagerstelle dienen, leicht über diese Stelle hinweg geführt werden können und der Rotor beim Aufstecken nicht verhakt.

Besonders bevorzugt wird es, wenn der Kopf, die Nut und der Anlagebereich für den reibschlüssigen Antrieb rotationssymmetrisch sind. Damit wird erreicht, dass der Rotor in jeder beliebigen Position auf die Welle gesteckt werden kann und die korrekte Drehposition nicht erst gefunden werden muss.

Die Rotationseinheit gemäß der Offenbarung ist für eine peristaltische Blutpumpe ausgelegt und weist eine vorbeschriebene Antriebswelle und einen darauf ausgesteckten oder aufsteckbaren Rotor mit Schlauchrollen auf. Die Nut hat eine dem Kopf zugewandte und daher als kopfseitig bezeichnete Nutflanke, die senkrecht zur Mittelachse angeordnet ist und damit vorzugsweise die Form eines Kreisrings mit maximaler Steilheit hat. In dem Rotor ist ein Verriegelungselement angeordnet, das quer zur Mittelachse der Antriebswelle bewegbar ist. Ein Anlageabschnitt des Verriegelungselements ist mittels einer Feder radial in Richtung zur Mittelachse und in Richtung zum Nutgrund vorgespannt, wobei der Anlageabschnitt mittels eines am Verriegelungselement befestigten oder einstückig gebildeten Betätigungsabschnitts in Richtung radial weg von der Mittelachse und in Richtung weg vom Nutgrund bewegbar ist. Zumindest eine Anlagefläche des Anlageabschnitts oder der gesamte Anlageabschnitt ist so wie die kopfseitige Nutflanke senkrecht zur Mittelachse angeordnet. Wenn der Rotor mit einer Kraft weg vom Boden des Gehäuses in Abzugsrichtung des Rotors beaufschlagt wird, und wenn dabei nicht auf den Betätigungsabschnitt gedrückt wird, ergibt sich ein sicherer Halt des Rotors im Betrieb der Blutpumpe wegen der senkrechten Ausrichtung einerseits der kopfseitigen Nutflanke und andererseits der Anlagefläche des Anlageabschnitts.

Der Betätigungsabschnitt kann z.B. eine Betätigungsfläche sein, er kann aber auch eine abweichende z.B. ballige Form haben.

Vorzugsweise ist der Anlageabschnitt auf einer dem Betätigungsabschnitt gegenüberliegenden Seite der Antriebswelle angeordnet. Dann erzeugt ein Drücken des Betätigungsabschnitts hin zur Antriebswelle ein Herausfahren des Anlageabschnitts auf der anderen Seite der Antriebswelle. Dazu ist ein Überbrückungsabschnitt am Verriegelungselement nötig, der sich vorzugsweise einseitig an der Antriebswelle entlang erstreckt und den Betätigungsabschnitt vorzugsweise einstückig mit dem Anlageabschnitt verbindet.

Wenn die Feder eine Druckfeder ist, ist diese auch auf der dem Betätigungsabschnitt gegenüberliegenden Seite der Antriebswelle angeordnet.

Bei einer besonders bevorzugten Ausgestaltung des Verriegelungselements ist der Anlageabschnitt kreisbogenförmig und erstreckt sich in einem Umfangsbereich der Nut. Vorzugsweise erstreckt sich der Anlageabschnitt über 80° bis 96° entlang dem Umfang der Nut. Mit zunehmender Erstreckung in Richtung zum Betätigungsabschnitt verlängert sich der Weg des gesamten Verriegelungselements, der nötig ist, um dessen Anlageabschnitt komplett aus der Nut herauszubewegen. Damit ist die Sicherheit gegen ungewolltes Entriegeln des Rotors erhöht.

Bei einer vorrichtungstechnisch einfachen Ausgestaltung ist das Verriegelungselement mit dem Betätigungsabschnitt, dem Überbrückungsabschnitt und dem Anlageabschnitt ein gestanztes Blechteil. Dabei kann der Betätigungsabschnitt durch Abwinkeln bzw. Abkanten z.B. um 90° gebildet sein.

Bei einer bevorzugten Ausgestaltung hat der Betätigungsabschnitt einen geringeren Abstand zur Antriebswelle, als ein den Betätigungsabschnitt umgebender Gehäuseabschnitt des Rotors. Damit ist der Betätigungsabschnitt im Gehäuseabschnitt des Rotors versenkt, und die Sicherheit gegen ungewolltes Drücken und somit Entriegeln des Rotors ist erhöht.

Bei einer bevorzugten Ausgestaltung bewirkt die Feder eine Losbrechkraft, also eine Betätigungskraft am Betätigungselement ausgehend von einer Verriegelungsposition - von mindestens 10 N. Damit ist die Sicherheit gegen ungewolltes Entriegeln des Rotors gegenüber dem Stand der Technik erhöht. Mit zunehmender Losbrechkraft erhöht sich die Fingerkraft, die nötig ist, um den Anlageabschnitt komplett aus der Nut herauszubewegen. Damit kann die Sicherheit gegen ungewolltes Entriegeln des Rotors weiter erhöht werden.

Eine Entnahmekraft für den Rotor 1 beträgt mindestens 12 N.

### Kurzbeschreibung der Figuren

Figur 1 ist eine peristaltische Blutpumpe mit einer Rotationseinheit gemäß dem Ausführungsbeispiel der vorliegenden Offenbarung;
Figur 2 ist eine geschnittene Darstellung der Rotationseinheit aus Figur 1;
Figur 3 ist ein Ausschnitt der Rotationseinheit aus Figur 1 in einer weiteren geschnittenen Darstellung mit zwei Varianten eines Anlageabschnitts;
Figur 4 ist eine Ansicht des für die Offenbarung wesentlichen Teils der Antriebswelle aus den vorhergehenden Figuren.

### Beschreibung des Ausführungsbeispiels

Nachstehend wird ein Ausführungsbeispiel der Rotationseinheit mit einem Ausführungsbeispiel der Antriebswelle auf der Basis der zugehörigen Figuren beschrieben.

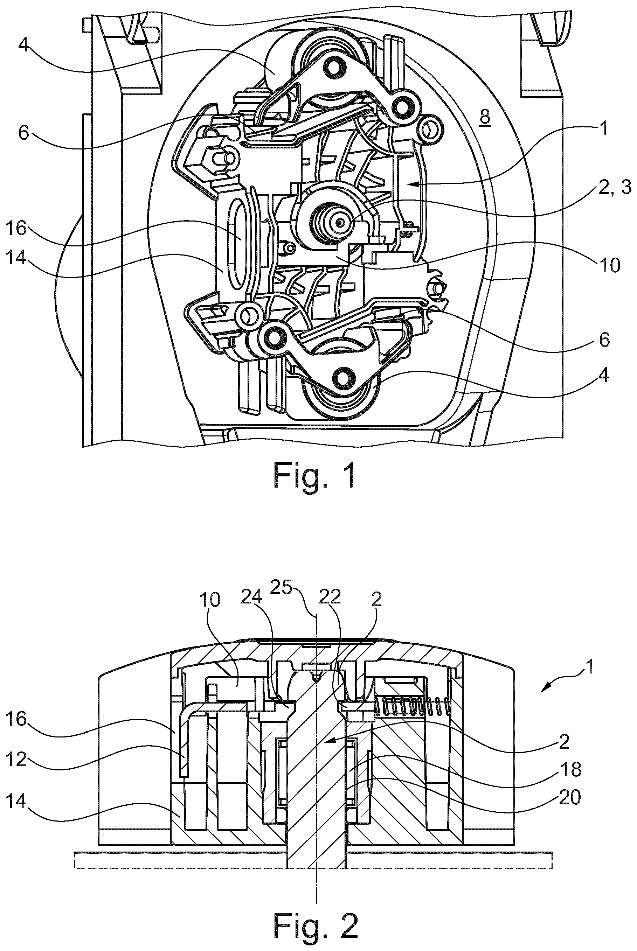

Figur 1 ist eine peristaltische Blutpumpe mit einer Rotationseinheit gemäß dem Ausführungsbeispiel der vorliegenden Offenbarung. Die Rotationseinheit weist einen Rotor 1 und eine zapfenartige Antriebswelle 2 auf, von der (in Figur 1) nur der Kopf 3 zu erkennen ist.

Der Rotor 1 hat an seinem Umfang verteilt zwei Rollen 4, die über die Kraft eine jeweiligen Andruckfeder 6 nach außen in Richtung zu einer zumindest in einem Mittelbereich kreisbogenförmigen bahnartigen Lauffläche 8 gespannt sind. An der Lauffläche 8 wir ein Schlauchsegment eines (nicht gezeigten) Schlauches angelegt. Das zu fördernde Blut wird durch das an der Lauffläche 8 anliegende Schlauchsegment gefördert, in dem die Rollen 4 das Schlauchsegment eindrücken und die eingedrückte Stelle gemäß der Rotation des Rotors 1 an dem Schlauchsegment entlang bewegt wird.

Da in Figur 1 ein Gehäusedeckel des Rotors 1 entfernt ist, ist der Kopf 3 der Antriebswelle 2 zu erkennen. Der Rotor 1 wird in axialer Richtung an der Antriebswelle 2 mit einem als Verriegelungsblech ausgeführten Verriegelungselement 10 gehalten. Dies hat einen als Betätigungsfläche ausgebildeten Betätigungsabschnitt 12, der radial innerhalb eines Gehäuseabschnitts 14 des Rotors 1 angeordnet ist. Durch eine Durchgangsausnehmung 16 des Gehäuseabschnitts 14 kann der Betätigungsabschnitt 12 vom Bedienpersonal gedrückt werden, um die axiale Verriegelung zu lösen und den Rotor 1 von der Antriebwelle 2 abzuziehen.

Figur 2 ist eine geschnittene Darstellung der Rotationseinheit aus Figur 1. Der Rotor 1 ist über eine reibschlüssige Kupplung 18 mit einem kreiszylindrischen Anlagebereich 20 der Antriebswelle 2 in einer Förderrichtung drehfest verbunden, während in Gegenrichtung ein Freilauf besteht.

Das Verriegelungselement 10 ist einstückig als Stanz-Biege-Blechteil hergestellt und hat einen Anlageabschnitt 22, der in eine umlaufende Nut 24 der zur Mittelachse 25 rotationsymmetrischen Antriebswelle 2 eintaucht, um ein axiales Abziehen des Rotors 1 (in Figur 2 nach oben) zu verhindern.

Das Verriegelungselement 10 hat den durch Biegen erzeugten Betätigungsabschnitt 12, der als Betätigungsfläche ausgebildet ist, und der auch als Druckknopf angesehen werden kann. Der Betätigungsabschnitt 12 ist gegenüber dem Gehäuseabschnitt 14 radial zurückgesetzt. Damit ist eine versehentliche Betätigung und somit Lösen des Rotors 1 vermeiden. Der Betätigungsabschnitt 12 ist etwa parallel zur Mittelachse 25 ausgerichtet. Damit kann der Finger, mit dem der Rotor 1 gelöst wird, auch zum einhändigen Herausnehmen des Rotors 1 genutzt werden. Für einen sicheren Halt des Fingers am Rotor 1 dient auch die Durchgangsausnehmung 16 im Gehäuseabschnitt 14.

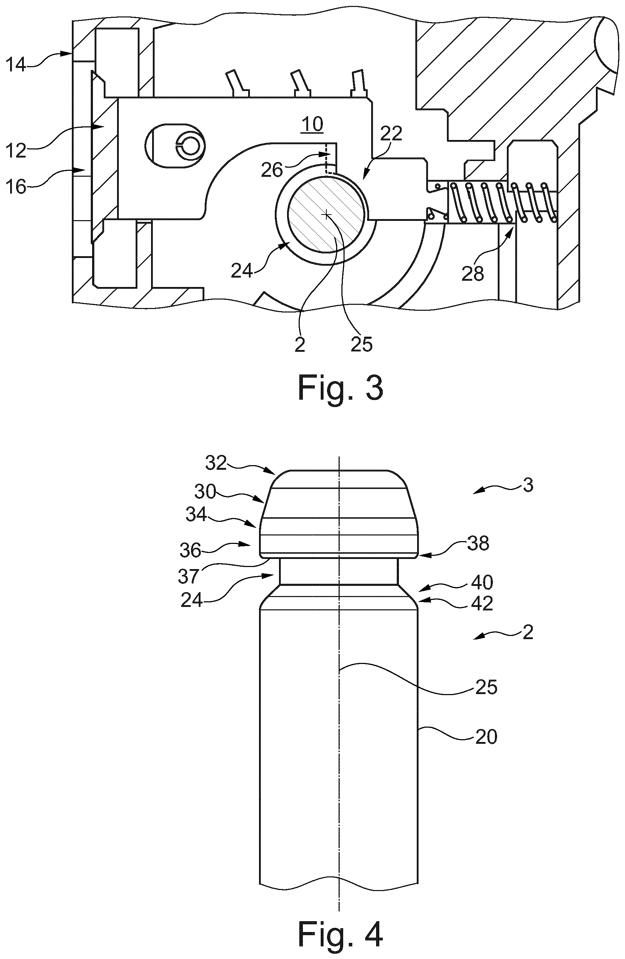

Figur 3 ist ein Ausschnitt der Rotationseinheit aus Figur 1 in einem Querschnitt durch die Antriebswelle 2. Insbesondere ein Hauptabschnitt des Verriegelungselement 10 ist in einer Ansicht (von unten) gezeigt, und der Betätigungsabschnitt 12 ist in seinem abgewinkelten Übergang zum Hauptabschnitt des Verriegelungselements 10 geschnitten dargestellt.

Das Verriegelungselement 10 umgreift die Antriebswelle 2 einseitig, in anderen Worten ausgedrückt hat das Verriegelungselement 10 eine abschnittsweise konkave Ausnehmung, in der die Antriebswelle 2 angeordnet ist. Am Rand dieser Ausnehmung ist ein kreisbogenförmiger Anlageabschnitt 22 gebildet, der in Richtung zur Nut 24 der Antriebswelle 2 mittels einer Feder 28 gespannt ist. die Feder 28 ist auf einer bezüglich der Antriebwelle 2 dem Betätigungsabschnitt 12 gegenüberliegenden Seite angeordnet und als Druckfeder ausgebildet. Die Feder 28 bewirkt eine Losbrechkraft am Betätigungselement 12 von mindestens 10 N, und eine Entnahmekraft für den Rotor 1 beträgt mindestens 12 N. Damit ist eine hohe Sicherheit gegen ungewolltes Entriegeln und Lösen des Rotors 1 gegeben.

In durchgezogener Linie ist eine kleinere Variante des Anlageabschnitts 22 dargestellt. Diese Variante bzw. dieser Anlageabschnitt 22 erstreckt sich etwa 82° um die Nut 24 bzw. die Antriebswelle 2 herum.

Der Weg, der zum Herausbewegen des Anlageabschnitts 22 nötig ist, kann vergrößert werden, wenn am Anlageabschnitt 22 eine optionale Vergrößerung 26 vorgesehen wird (gepunktet dargestellt). Diese Variante erstreckt sich etwa 90° um die Nut 24 bzw. die Antriebswelle 2 herum.

Figur 4 ist eine Ansicht des Teils der Antriebswelle 2 aus den vorhergehenden Figuren auf den der Rotor 1 ausgesteckt wird. Dieser Teil der Antriebswelle 2 erstreckt sich entlang ihrer Mittelachse 25 und weist an einem freien Endabschnitt den Kopf 3 auf, der von der - entlang der Mittelachse 25 betrachtet benachbarten - Nut 24 begrenzt ist. An dem Kopf 3 ist ein durch eine Fase gebildeter konischer oder kegelstumpfförmiger Gleitabschnitt 30 angeordnet. Damit ist die Montagekraft für den Rotor 1 gering. Damit ist auch eine Einführhilfe für die Wellenaufnahme des Rotors 1 geschaffen, die einen axialen Versatz und eine nicht senkrechte Aufsteckbewegung toleriert und ein Verhaken des Rotors 1 vermeidet.

Der Gleitabschnitt 30 erstreckt sich - entlang der Mittelachse 25 der Antriebswelle betrachtet - über etwa 35% der Axialerstreckung (der Länge) des Kopfes 3 und hat einen Winkel von 15° zur Mittelachse 25 der Antriebswelle 3.

An der von der von der Nut 24 abgewandten Seite der Fase weist der Kopf 3 eine Stirnfläche auf, wobei ein Übergang von der Fase bzw. dem Gleitabschnitt 30 zu der Stirnfläche verrundet ist mit einem Radius 32 von etwa 1,5 mm. Dadurch ist das Aufstecken des Rotors 1 weiter vereinfacht und ein Verhaken vermieden.

Zwischen der Fase bzw. dem Gleitabschnitt 30 und der Nut 24 ist ein kreiszylindrischer Kopfabschnitt 36 vorgesehen, der sich - entlang der Mittelachse 25 der Antriebswelle 2 betrachtet - über etwa 20% der Axialerstreckung (der Länge) des Kopfes 3 erstreckt. Ein Übergang von dem Gleitabschnitt 30 zu dem kreiszylindrischen Kopfabschnitt 36 ist verrundet mit einem Radius 34 von 4 mm. Damit ist das Aufstecken des Rotors 1 weiter vereinfacht und ein Verhaken vermieden.

Ein Übergang von dem kreiszylindrischen Kopfabschnitt 36 zur kopfseitigen Nutflanke 37 der Nut 24 ist verrundet mit einem Radius 38 von 0,25 mm. Diese Verrundung 38 dient hauptsächlich einem einfacheren Entnehmen des Rotors 1, damit dieser während der Entnahme nicht so leicht verhakt. Die Verrundung bzw. der Radius 38 ist vergleichsweise klein, damit kein großer Einfluss auf den Hinterschnitt des Verriegelungselements 10 entsteht.

An einer von dem Kopf 3 abgewandten Seite der Nut 24 ist der kreiszylindrische Anlagebereich 20 der reibschlüssigen Kupplung 18 gebildet. Eine zwischen einem Nutgrund der Nut 24 und dem kreiszylindrischen Anlagebereich 20 angeordnete antriebsseitige Nutflanke 40 ist konisch oder kegelstumpfförmig zur Mittelachse 25 schräg gestellt. Ein Übergang 42 von der Nutflanke 40 zu dem kreiszylindrischen Anlagebereich 20 ist verrundet mit einem Radius 42 von 2 mm. Damit wird erreicht, dass die Passungen im Rotor 1, die als Lagerstelle dienen, leicht über den Übergang 42 hinweg geführt werden können und der Rotor 1 beim Aufstecken nicht verhakt.

Zusammenfassend ist in den vorstehenden Ausführungsbeispielen also ein Peristaltikpumpe gezeigt, deren Antriebswelle 2 rotationssymmetrisch ist, so dass ein aufzusteckender Rotor 1 ohne Druck auf den Betätigungsabschnitt 12 und ohne Einhaltung einer vorbestimmte Drehposition aufgesteckt werden kann. Dabei werden wegen des durch eine Fase gebildeten konischen oder kegelstumpfförmigen Gleitabschnitts 30 am Kopf 3 der Antriebwelle 2 auch Abweichungen von der idealen Aufsteckrichtung toleriert.

Um ein versehentliches Lösen des Rotors 1 von der Antriebswelle 2 zu vermeiden kann ergänzend zumindest eine der folgenden Maßnahmen vorgesehen werden:
- Der Betätigungsabschnitt 12 (Druckknopf) ist gegenüber dem Gehäuseabschnitt 14 des Rotors 1 versenkt.
- Die Feder 28 ist derart ausgelegt, dass der Betätigungsabschnitt 12 eine Losbrechkraft von 10N hat.
- der Anlageabschnitt 22 (mit seiner Anlagefläche für die kopfseitige Nutflanke) erstreckt sich auf einem Kreisbogen um 90° um die Nut 24 herum.

### Bezugszeichenliste:

- 1: Rotor
- 2: Antriebswelle
- 3: Kopf
- 4: Rolle
- 6: Andruckfeder
- 8: Lauffläche
- 10: Verriegelungselement
- 12: Betätigungsabschnitt
- 14: Gehäuseabschnitt
- 16: Durchgangsausnehmung
- 18: Kupplung
- 20: Anlagebereich (der Antriebswelle)
- 22: Anlageabschnitt (des Verriegelungselements)
- 24: Nut
- 25: Mittelachse
- 26: (optionale) Vergrößerung des Anlageabschnitts (des Verriegelungselements)
- 28: Feder
- 30: Gleitabschnitt
- 32: Übergang / Radius
- 34: Übergang / Radius
- 36: kreiszylindrischer Kopfabschnitt
- 37: kopfseitige Nutflanke
- 38: Übergang / Radius
- 40: antriebsseitige Nutflanke
- 42: Übergang / Radius

## Patentansprüche

1. Antriebswelle (2) für eine peristaltische Blutpumpe, die zum Aufstecken eines Rotors (1) der Blutpumpe eingerichtet und ausgelegt ist, wobei sich die Antriebswelle (2) entlang einer Mittelachse (25) erstreckt und an einem freien Endabschnitt einen rotationssymmetrischen Kopf (3) hat, der von einer Nut (24) begrenzt ist, wobei die Nut (24) vorzugsweise eine kopfseitige Nutflanke (37) hat, die senkrecht zur Mittelachse (25) angeordnet ist, **dadurch gekennzeichnet, dass** an dem Kopf (3) ein durch eine Fase gebildeter konischer oder kegelstumpfförmiger Gleitabschnitt (30) angeordnet ist.

2. Antriebswelle (2) nach Anspruch 1 **dadurch gekennzeichnet, dass** sich der Gleitabschnitt (30) entlang der Mittelachse (25) betrachtet über 30 bis 40% der Axialerstreckung des Kopfes (3) erstreckt.

3. Antriebswelle (2) nach einem der vorhergehenden Ansprüche **dadurch gekennzeichnet, dass** der Gleitabschnitt (30) einen Winkel in einem Bereich von 5° bis 20° zur Mittelachse (25) der Antriebswelle (2) hat.

4. Antriebswelle (2) nach einem der vorhergehenden Ansprüche, wobei der Kopf (3) eine Stirnfläche hat, **dadurch gekennzeichnet, dass** ein Übergang (32) von der Stirnfläche zu dem Gleitabschnitt (30) verrundet ist, vorzugsweise mit einem Radius (32) von größer als 1 mm.

5. Antriebswelle (2) nach einem der vorhergehenden Ansprüche **dadurch gekennzeichnet, dass** zwischen dem Gleitabschnitt (30) und der Nut (24) ein kreiszylindrischer Kopfabschnitt (36) vorgesehen ist.

6. Antriebswelle (2) nach Anspruch 5 **dadurch gekennzeichnet, dass** der kreiszylindrische Kopfabschnitt (36) entlang der Mittelachse (25) der Antriebswelle (2) betrachtet in einem Bereich von 15 bis 25% der Axialerstreckung des Kopfes (3) erstreckt.

7. Antriebswelle (2) nach Anspruch 5 oder 6 **dadurch gekennzeichnet, dass** ein Übergang (34) von dem Gleitabschnitt (30) zu dem kreiszylindrischen Kopfabschnitt (36) verrundet ist vorzugsweise mit einem Radius (34) in einem Bereich von 2 bis 6 mm.

8. Antriebswelle (2) nach einem der Ansprüche 5 bis 7 **dadurch gekennzeichnet, dass** ein Übergang (38) von dem kreiszylindrischen Kopfabschnitt (36) zu der Nut (24) verrundet ist vorzugsweise mit einem Radius (38) in einem Bereich von 0,1 bis 0,3 mm.

9. Antriebswelle (2) nach einem der vorhergehenden Ansprüche, wobei an einer von dem Kopf (3) abgewandten Seite der Nut (24) ein kreiszylindrischer Anlagebereich (20) gebildet ist, wobei eine zwischen einem Nutgrund und dem kreiszylindrischen Anlagebereich (20) angeordnete antriebsseitige Nutflanke (40) konisch oder kegelstumpfförmig ist, **dadurch gekennzeichnet, dass** ein Übergang (42) von der antriebsseitigen Nutflanke (40) zu dem kreiszylindrischen Anlagebereich (20) verrundet ist vorzugsweise mit einem Radius (42) in einem Bereich zwischen 1,5 und 4 mm.

10. Rotationseinheit für eine peristaltische Blutpumpe mit einer Antriebswelle (2) gemäß einem der vorhergehenden Ansprüche und mit einem darauf ausgesteckten oder aufsteckbaren Rotor (1) mit Schlauchrollen (4), wobei die Nut (24) eine kopfseitige Nutflanke (37) hat, die senkrecht zur Mittelachse (25) angeordnet ist, und wobei in dem Rotor (1) ein Verriegelungselement (10) angeordnet ist, das quer zur Mittelachse (25) bewegbar ist, und dessen Anlageabschnitt (22) mittels einer Feder (28) in Richtung zur Mittelachse (25) und in Richtung zum Nutgrund vorgespannt ist, wobei der Anlageabschnitt (22) mittels eines am Verriegelungselement (10) befestigten oder einstückig gebildeten Betätigungsabschnitts (12) in Richtung weg von der Mittelachse (25) und in Richtung weg vom Nutgrund bewegbar ist, **dadurch gekennzeichnet, dass** eine Anlagefläche des Anlageabschnitts (22) senkrecht zur Mittelachse (25) angeordnet ist.

11. Rotationseinheit nach Anspruch 10 **dadurch gekennzeichnet, dass** der Anlageabschnitt (22) auf einer dem Betätigungsabschnitt (12) gegenüberliegenden Seite der Antriebswelle (2) angeordnet ist.

12. Rotationseinheit nach Anspruch 11 **dadurch gekennzeichnet, dass** der Anlageabschnitt (22) kreisbogenförmig ist und sich in einem Umfangsbereich der Nut (24) vorzugsweise über 75° bis 95° erstreckt.

13. Rotationseinheit nach einem der Ansprüche 10 bis 12 **dadurch gekennzeichnet, dass** der Betätigungsabschnitt (12) einen geringeren Abstand zur Antriebswelle (2) hat, als ein den Betätigungsabschnitt (12) umgebender Gehäuseabschnitt (14) des Rotors (1).

14. Rotationseinheit nach einem der Ansprüche 10 bis 13 **dadurch gekennzeichnet, dass** das Verriegelungselement (10) - ausgehend von einer Verriegelungsposition - eine Losbrechkraft von mindestens 10 N hat.

## Claims

1. A drive shaft (2) for a peristaltic blood pump which is arranged and designed for attaching a rotor (1) of the blood pump, wherein the drive shaft (2) extends along a central axis (25) and, at a free end section, has a rotationally symmetric head (3) which is delimited by a groove (24), the groove (24) preferably including a head-side groove flank (37) which is arranged perpendicularly to the central axis (25), **characterized in that** a conical or frustoconical sliding section (30) formed by a chamfer is arranged on the head (3).

2. The drive shaft (2) according to claim 1, **characterized in that** the sliding section (30) extends, viewed along the central axis (25), over 30 to 40% of the axial extension of the head (3).

3. The drive shaft (2) according to any one of the preceding claims, **characterized in that** the sliding section (30) has an angle in a range from 5° to 20° to the central axis (25) of the drive shaft (2).

4. The drive shaft (2) according to any one of the preceding claims, wherein the head (3) has a front face, **characterized in that** a transition (32) from the front face to the sliding section (30) is rounded, preferably with a radius (32) of more than 1 mm.

5. The drive shaft (2) according to any one of the preceding claims, **characterized in that** a circular cylindrical head section (36) is provided between the sliding section (30) and the groove (24).

6. The drive shaft (2) according to claim 5, **characterized in that** the circular cylindrical head section (36) extends, viewed along the central axis (25) of the drive shaft (2), in a range from 15 to 25% of the axial extension of the head (3).

7. The drive shaft (2) according to claim 5 or 6, **characterized in that** a transition (34) from the sliding section (30) to the circular cylindrical head section (36) is rounded, preferably with a radius (34) in a range from 2 to 6 mm.

8. The drive shaft (2) according to any one of the claims 5 to 7, **characterized in that** a transition (38) from the circular cylindrical head section (36) to the groove (24) is rounded, preferably with a radius (38) in a range from 0.1 to 0.3 mm.

9. The drive shaft (2) according to any one of the preceding claims, wherein a circular cylindrical abutment area (20) is formed on a side of the groove (24) remote from the head (3), wherein a drive-side groove flank (40) interposed between a groove bottom and the circular cylindrical abutment area (20) is conical or frustoconical, **characterized in that** a transition (42) from the drive-side groove flank (40) to the circular cylindrical abutment area (20) is rounded, preferably with a radius (42) in a range between 1.5 and 4 mm.

10. A rotation unit for a peristaltic blood pump comprising a drive shaft (2) according to any one of the preceding claims, and comprising a rotor (1) including tube rollers (4) which is or can be attached to said drive shaft (2), wherein the groove (24) has a head-side groove flank (37) that is arranged perpendicularly to the central axis (25), and wherein a locking element (10) which is movable transversely to the central axis (25) and the abutment section (22) of which is pretensioned in the direction of the central axis (25) and in the direction of the groove bottom by means of a spring (28) is arranged in the rotor (1), wherein the abutment section (22) is movable in the direction away from the central axis (25) and in the direction away from the groove bottom by means of an actuating section (12) mounted on or formed integrally with the locking element (10), **characterized in that** an abutment surface of the abutment section (22) is arranged perpendicularly to the central axis (25).

11. The rotation unit according to claim 10, **characterized in that** the abutment section (22) is arranged on a side of the drive shaft (2) opposite to the actuating section (12).

12. The rotation unit according to claim 11, **characterized in that** the abutment section (22) is circular arc-shaped and extends in a circumferential area of the groove (24) preferably over 75° to 95°.

13. The rotation unit according to any one of the claims 10 to 12, **characterized in that** the actuating section (12) has a smaller distance from the drive shaft (2) than a housing section (14) of the rotor (1) surrounding the actuating section (12).

14. The rotation unit according to any one of the claims 10 to 13, **characterized in that** - starting from a locking position - the locking element (10) has a breakaway force of at least 10 N.

## Revendications

1. Arbre d'entraînement (2) pour une pompe à sang péristaltique qui est conçue et configurée pour l'enfichage d'un rotor (1) de la pompe à sang, dans lequel l'arbre d'entraînement (2) s'étend le long d'un axe central (25) et présente au niveau d'une section d'extrémité libre une tête (3) symétrique en rotation qui est délimitée par une rainure (24), dans lequel la rainure (24) présente de préférence un flanc de rainure (37) côté tête qui est disposé perpendiculairement à l'axe central (25), **caractérisé en ce qu'**une section coulissante (30) tronconique ou conique formée par un chanfrein est agencée au niveau de la tête (3).

2. Arbre d'entraînement (2) selon la revendication 1, **caractérisé en ce que** la section coulissante (30) s'étend le long de l'axe central (25) considérée sur 30 à 40 % de l'étendue axiale de la tête (3).

3. Arbre d'entraînement (2) selon l'une quelconque des revendications précédentes, **caractérisé en ce que** la section coulissante (30) présente un angle dans une plage de 5° à 20° par rapport à l'axe central (25) de l'arbre d'entraînement (2).

4. Arbre d'entraînement (2) selon l'une quelconque des revendications précédentes, dans lequel la tête (3) présente une surface avant, **caractérisé en ce qu'**une transition (32) de la surface avant à la section coulissante (30) est arrondie, de préférence avec un rayon (32) de plus de 1 mm.

5. Arbre d'entraînement (2) selon l'une quelconque des revendications précédentes, **caractérisé en ce qu'**une section de tête (36) cylindrique circulaire est prévue entre la section coulissante (30) et la rainure (24).

6. Arbre d'entraînement (2) selon la revendication 5, **caractérisé en ce que** la section de tête (36) cylindrique circulaire s'étend le long de l'axe central (25) de l'arbre d'entraînement (2) considérée dans une plage de 15 à 25 % de l'étendue axiale de la tête (3).

7. Arbre d'entraînement (2) selon la revendication 5 ou 6, **caractérisé en ce qu'**une transition (34) de la section coulissante (30) à la section de tête (36) cylindrique circulaire est arrondie, de préférence avec un rayon (34) dans une plage de 2 à 6 mm.

8. Arbre d'entraînement (2) selon l'une quelconque des revendications 5 à 7, **caractérisé en ce qu'**une transition (38) de la section de tête (36) cylindrique circulaire à la rainure (24) est arrondie, de préférence avec un rayon (38) dans une plage de 0,1 à 0,3 mm.

9. Arbre d'entraînement (2) selon l'une quelconque des revendications précédentes, dans lequel une zone d'appui (20) cylindrique circulaire est formée au niveau d'un côté éloigné de la tête (3) de la rainure (24), dans lequel un flanc de rainure (40) côté entraînement agencé entre un fond de rainure et la zone d'appui (20) cylindrique circulaire est conique ou tronconique, **caractérisé en ce qu'**une transition (42) du flanc de rainure (40) côté entraînement à la zone d'appui (20) cylindrique circulaire est arrondie, de préférence avec un rayon (42) dans une plage entre 1,5 et 4 mm.

10. Unité de rotation pour une pompe à sang péristaltique avec un arbre d'entraînement (2) selon l'une quelconque des revendications précédentes et avec un rotor (1) enfiché ou enfichable dessus avec des rouleaux de tuyau (4), dans laquelle la rainure (24) présente un flanc de rainure (37) côté tête qui est agencé perpendiculairement à l'axe central (25), et dans laquelle un élément de verrouillage (10) est agencé dans le rotor (1), élément qui est mobile transversalement à l'axe central (25), et dont la section d'appui (22) est précontrainte au moyen d'un ressort (28) en direction de l'axe central (25) et en direction du fond de rainure, dans laquelle la section d'appui (22) est mobile au moyen d'une section d'actionnement (12) formée d'un seul tenant ou fixée à l'élément de verrouillage (10) dans une direction s'éloignant de l'axe central (25) et dans une direction s'éloignant du fond de rainure, **caractérisée en ce qu'**une surface d'appui de la section d'appui (22) est agencée perpendiculairement à l'axe central (25).

11. Unité de rotation selon la revendication 10, **caractérisée en ce que** la section d'appui (22) est agencée sur un côté opposé à la section d'actionnement (12) de l'arbre d'entraînement (2).

12. Unité de rotation selon la revendication 11, **caractérisée en ce que** la section d'appui (22) est en forme d'arc de cercle et s'étend dans une zone périphérique de la rainure (24), de préférence sur 75° à 95°.

13. Unité de rotation selon l'une quelconque des revendications 10 à 12, **caractérisée en ce que** la section d'actionnement (12) présente une distance inférieure par rapport à l'arbre d'entraînement (2) qu'une section de boîtier (14) entourant la section d'actionnement (12) du rotor (1).

14. Unité de rotation selon l'une quelconque des revendications 10 à 13, **caractérisée en ce que** l'élément de verrouillage (10) présente, à partir d'une position de verrouillage, une force de décollement d'au moins 10 N.
